# EUROPEAN PATENT APPLICATION

(11) **EP 3 278 817 A1**
(43) Date of publication of application: **07.02.2018**
(21) Application number: 16771446.8
(22) Date of filing: 29.03.2016
(51) Int. Cl.: A61L 2/06, G05D 23/19

(54) **METHOD FOR THE DECONTAMINATION AND DISINFECTION OF A TRANSPORT VEHICLE, AND DEVICE FOR CONTROLLING SAID DECONTAMINATION**

(30) Priority: 30.03.2015 ES 201530422; 30.03.2015 ES 201530366 U
(71) Applicant: Castañe Basagaña, Sebastian, 08572 Sant Pere De Torello (Barcelona) (ES)
(72) Inventor: Castañe Basagaña, Sebastian, 08572 Sant Pere De Torello (Barcelona) (ES)
(74) Representative: Mohammadian, Dario
(86) International application number: PCT/ES2016/070214
(87) International publication number: WO 2016/156645

(57) **Abstract**

Method for the decontamination and disinfection of a transport vehicle, and control device for said decontamination. The method according to the invention comprises at least one heat treatment phase of the vehicle cabin, or of at least one cabin compartment, intended for transporting, by injecting a hot gas into the same at a temperature that guarantees the elimination of the pathogens, said temperature being comprised between 0°C and 91°C. The control device for the decontamination comprises: a control unit (1); a user interface (2); a series of conveniently distributed temperature sensors (3); and a memory (5) for the storage of data relating to dates, temperatures and other variables relating to the decontaminations carried out, as well as the identification of the vehicle on which the decontamination process is carried out.

## Description

### Object of the invention.

The present invention refers to a method for the decontamination and disinfection of the transport cabin, or at least a compartment of the cabin, of a motor vehicle or similar, in particular for the microbiologic deodorization permitting the elimination of the bad odors after having transported animals or contaminant products in the vehicle, as well as contamination produced especially by diverse pathogens, such as: salmonella, diphtheria, PED (Porcine epidemic diarrhea), etc. after transporting live animals in the truck prepared for such purpose.

This invention includes also a device destined for controlling the method of disinfection and decontamination realized.

### Background art

Currently several methods for the disinfection and decontamination of motor vehicles are known. Most of them are applied after washing the vehicle and use chemical products or substances of different types, such as foam or vapors, which are normally applied by means of aerosols or vaporizers. These products have a limited efficacy, as they usually utilize active ingredients of reduced anti-bacterial effect; on the other hand, they have little or no deodorizing effect as they normally simply cover odors instead of permanently eliminating them, and they suffer the drawback of leaving, after the treatment, chemical residues which in some cases are very harmful and are not easy to eliminate.

Currently other known solutions combine several chemical agents which provoke an exothermic reaction that generates vapors capable of realizing a disinfecting action. These solutions are effective, however are difficult to execute as it becomes necessary to combine different chemical substances before the treatment and they produce generally toxic chemical residues that are not easy to eliminate.

In relation to equipment used for vehicle decontamination, for example described in document ES-10677576, they consist of a container situated on top of the load cabin of a truck from which the decontamination work is performed, in which the autonomous equipment, rollers, hoses, connections, flexible water deposits (clean and residual), suction pumps, residual water collection trays, decontamination rods, illumination, terrain bar, mixer, shower equipment for personnel decontamination, personnel protection suit wardrobe, decontamination portable equipment, and other accessories, are placed. Document ES-1067577 describes another autonomous decontamination equipment even more complex than the former.

Decontamination processes are nowadays performed in "ad hoc" installations, in which the vehicle is introduced and a long treatment process is followed, using chemical products or substances of differing types, such as foams or vapors, which are normally applied via aerosols or vaporizers. These installations issue a disinfection and decontamination certificate which includes the vehicle identification data (license plate), execution date, duration, temperatures and products utilized, etc.

The problem arises when setting up a decontamination equipment in the vehicle itself, or when it is possible to have small equipment, easily installable in a gasoline station or in the transport company itself, permitting performing the decontamination process autonomously by the vehicle's driver himself or any other operator knowing its functioning. In these cases it is also necessary to have a document indicating when and how the load compartment decontamination was performed and this is problematic as these types of installations do not have any means of issuing a certificate that this process has been performed. This documentation can be required for example by the consignee company of the next transport, to make sure its product will not be contaminated during the transport with the prior product.

### Description of the invention.

The object of the present invention is solving the afore-mentioned problems of the prior art by providing a method for the decontamination and microbiological deodorization of the load cabin, or of areas or compartments, of vehicles intended for transporting merchandise or live animals, which permits in particular microbiological decontamination, especially of diverse pathogen germs, such as: salmonella, diphtheria, PED (Porcine epidemic diarrhea), etc. and also eliminate bad odors after transporting live animals or contaminant products, all performed with a small number of simple operations which can be performed even by a single operator.

The method of the present invention permits disinfecting and microbiologically decontaminating the load zones of a transport vehicle without leaving toxic residues therein after treatment, with guarantees of efficacy in terms of results and security of use by the operator, and economically for a competitive price.

The method of transport vehicle decontamination and disinfection object of the invention comprises at least one heat treatment phase of the vehicle cabin, or of at least one compartment of the cabin, intended for transporting, by injecting a hot gas into the same at a temperature that guarantees the elimination of the pathogens. The temperature of the gas utilized in this phase of thermic treatment is comprised between 0°C and 91°C and optimally this temperature is above 45 °C.

Preferentially the gas used in the decontamination/disinfection of the cabin, or cabin compartment, is hot air, although ozone or a mix of both may also be used.

The decontamination/disinfection of the cabin, or cabin compartment, is performed by introducing hot air inside this enclosure, which has been previously adequately closed, in which case the gas is introduced therein through a duct, stemming from an external autonomous heating equipment. Optionally a jet of hot air can also be manually applied, which comes out of a nozzle in one end of a hose which conducts it from the heating apparatus to the vehicle point to be decontaminated.

Optionally, the air used in the decontamination/disinfection of the load cabin, or cabin compartment, incorporates a disinfecting product and/or a deodorizing product.

Therefore, this process enables performing the decontamination of the cabin of a vehicle by means of a thermic treatment of the vehicle cabin, or at least a compartment of the cabin, intended for transporting, by injecting hot air into the same at a temperature that guarantees the elimination of the pathogens. This process is performed using a simple functioning heating equipment, as it can be easily operated by the vehicle's driver itself. This equipment can be autonomous and be placed in a reduced space, within, for example, of a gasoline station area, of a consignee company, or even on top of the vehicle itself.

Another object of the present invention is therefore providing a device that permits controlling such an equipment and more importantly, facilitates the deliveryman, or whomever who might need it, the data relating to the dates and conditions in which the decontaminations were performed.

The transport vehicle decontamination control device object of the invention is an autonomous equipment, commanded by a control unit in charge of executing a dedicated software adequate for executing the functions which define the decontamination method, of receiving, from sensors placed in the vehicle, a series of data relating to the decontamination process, as well as storing, processing and transmitting such data, by electronic means, to the user of the system or to the vehicle's cosignatory company. Further, said control unit connects with a user interface, through which it manages instructions relating to the functioning of the method of decontamination, and presents to the user operations performed and, according to the circumstances, data relating to the executed process.

A series of temperature, humidity, etc. sensors, conveniently distributed in different points of the load compartment to be decontaminated, transmit, to the control unit, their readings, which are stored and/or processed during and at the end of the decontamination process. The data relating to the dates, temperatures, and other variables relating to the decontaminations performed, as well as the identification of the vehicle to which the decontamination is performed, is stored in a memory.

Preferentially, the device incorporates also a printer which permits printing proof of the decontaminating processes performed.

Further, an emitter/receiver is arranged permitting transmitting via Internet and downloading the data of the effected decontaminations of the corresponding vehicle in a mobile terminal, or in the information centre of all of the vehicle's fleet.

Optionally, this device incorporates additionally a USB port to connect an external data storage unit, or through which a management and/or control unit updating software is executed.

### Description of the figures

To complement the description and with the objective of facilitating the comprehension of the features of the invention, a set of drawings is annexed to the current specification in which, with illustrative and non-limiting character, the following has been represented:

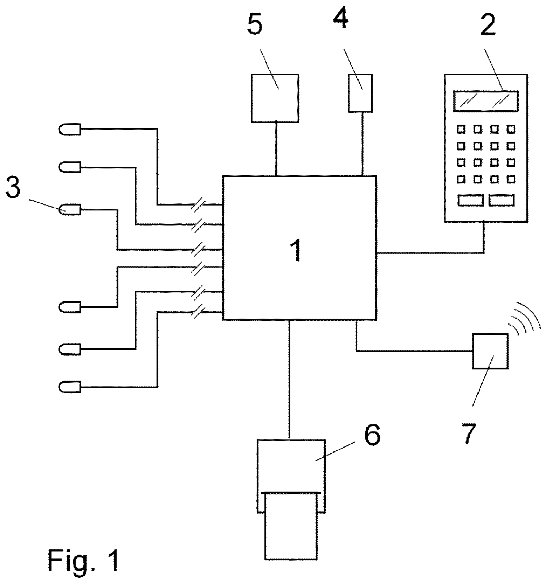

Figure 1 represents a functional block diagram of the device of the invention.

### Preferred embodiment of the invention

As can be appreciated from the figures, the device for controlling the decontamination of a transport vehicle is destined preferably to control the processes wherein the decontamination is performed by thermic treatment of the vehicle cabin, or at least a cabin compartment, intended for transporting, by injecting in the same a hot gas at a temperature that guarantees the elimination of the pathogens. This device is composed of the following essential elements or mechanisms:
a) A control unit (1), in charge of executing a software adequate for executing the functions which define the decontamination method executed by means of an autonomous equipment, or installed in the vehicle to be decontaminated itself, of receiving, from sensors (3) placed in the vehicle, a series of data relating to the decontamination process, as well as storing, processing and transmitting such data, by electronic means.
b) A user interface (2), which interacts with the control unit (1) to transmit to it the instructions relating to the functioning of the decontamination process, while concurrently being capable of presenting to the user the operations performed and, according to circumstances, the data relating to the process executed, as well as controlling the printing or transmitting by electronic means of the data relating to the performed decontamination process.
c) A series of temperature, or other parameter, sensors (3), conveniently distributed in different points of the load compartment to be decontaminated, connected to the control unit (1), which stores and/or processes its readings during and at the end of the process. These sensors preferably communicate via Wifi with the control unit (1).
d) A storage memory (5) for data relating to dates, temperatures, and other variables relating to the decontaminations performed, as well as the identification of the vehicle on which the decontaminating process is performed.

Additionally to these essential elements, the device optionally incorporates also a printer (6) by which the proof of the performed decontamination processes are printed.

Also optionally, this device incorporates an emitter/receiver that enables transmitting via Internet and downloading the data of the decontaminations performed on the corresponding vehicle, in a mobile terminal, or in the information centre of all of the vehicle's fleet.

Finally, and optionally, this device incorporates a USB port to connect an external unit for data storage, or for execution of control software of the control unit (1).

Once the nature of the invention has been sufficiently described, as well as a preferred embodiment, it is noted that the materials, forms, dimensions, and dispositions of the elements described may be modified, as long as the essential features of the invention described in the following are not altered:

## Claims

1. Method for the decontamination and disinfection of a transport vehicle, **characterized in** comprising at least one heat treatment phase of the vehicle cabin, or of at least one cabin compartment, intended for transporting, by injecting a hot gas into the same at a temperature that guarantees the elimination of the pathogens, said temperature being comprised between 0°C and 91°C.

2. Method according to claim 1, **characterized in that** the gas used in the decontamination/disinfection of the load cabin, or cabin compartment, is hot air.

3. Method according to the previous claims, **characterized in that** the gas used in the decontamination/disinfection of the load cabin, or cabin compartment, is hot at a temperature above 45°C.

4. Method according to the previous claims, **characterized in that** the decontamination/disinfection of the load cabin, or cabin compartment, is performed by introducing hot air within its enclosure, which has been previously adequately closed.

5. Method according to the previous claims, **characterized in that** the gas used in the decontamination/disinfection of the load cabin, or cabin compartment, is air which incorporates a disinfectant product.

6. Method according to the previous claims, **characterized in that** the gas used in the decontamination/disinfection of the load cabin, or cabin compartment, is air which incorporates a deodorizing product.

7. Method according to the previous claims, **characterized in that** the gas used in the decontamination/disinfection of the load cabin, or cabin compartment, provided by an external autonomous heating equipment, is introduced within the enclosure by means of a duct.

8. Device for controlling the decontamination of a transport vehicle, which is performed by means of a thermic treatment of the vehicle cabin, or at least a compartment of the cabin, intended for transporting, by injecting in the same a hot gas at a temperature which guarantees the elimination of pathogens, **characterized in** comprising:
a) a control unit (1), in charge of executing a software adequate for executing the functions which define the decontamination method executed by means of an autonomous equipment, or installed in the vehicle to be decontaminated itself, of receiving, from sensors placed in the vehicle, a series of data relating to the decontamination process, as well as storing, processing and transmitting such data, by electronic means;
b) a user interface (2), which interacts with the control unit (1) to transmit to it the instructions relating to the functioning of the decontamination process, as well as presenting to the user the operations performed and, according to circumstances, the data relating to the process executed, as well as its transmission by electronic means, or its printing;
c) a series of temperature sensors (3), conveniently distributed in different points of the load compartment to be thermally decontaminated, connected to the control unit (1), which stores and/or processes its readings during and at the end of the process;
d) a storage memory (5) for data relating to dates, temperatures, and other variables relating to the decontaminations performed, as well as the identification of the vehicle on which the decontaminating process is performed.

9. Device according to claim 8, **characterized in that** it incorporates also a printer (6) with which proofs of the performed decontamination processes are printed.

10. Device according to claims 8 and 9, **characterized in that** it incorporates additionally a emitter/receiver (7) which permits transmitting via Internet and downloading the data of the performed decontaminations of the corresponding vehicle in a mobile terminal, or in the information centre of the whole vehicle fleet.

11. Device according to claims 8 to 10, **characterized in that** it incorporates additionally a USB port for connecting an external unit for data storage, or for execution of a control software of the control unit (1).
